# EUROPÄISCHE PATENTSCHRIFT

(11) **EP 2 583 077 B1**
(45) Veröffentlichungstag und Bekanntmachung des Hinweises auf die Patenterteilung: **01.10.2014**
(21) Anmeldenummer: 11721229.0
(22) Anmeldetag: 21.04.2011
(51) Int. Cl.: G01N 1/08

(54) **HANDPRÜFGERÄT UND HANDPRÜFVERFAHREN ZUR BESCHAFFENHEITSUNTERSUCHUNG VON HOLZ**
MANUAL TESTING INSTRUMENT AND MANUAL TESTING METHOD FOR EXAMINING THE CONDITION OF WOOD
TESTEUR MANUEL ET PROCÉDÉ DE TEST MANUEL POUR L'ANALYSE DE L'ÉTAT DE BOIS

(30) Priorität: 23.04.2010 DE 102010018249
(43) Veröffentlichungstag der Anmeldung: 24.04.2013
(73) Patentinhaber: IML-Instrumenta Mechanik Labor GmbH, 69168 Wiesloch (DE)
(72) Erfinder: HUNGER, Erich, 76133 Karlsruhe (DE); HUNGER, Sebastian, 69181 Leimen (DE)
(74) Vertreter: Straub, Bernd
(86) Internationale Anmeldenummer: PCT/EP2011/002039
(87) Internationale Veröffentlichungsnummer: WO 2011/131363

(56) Entgegenhaltungen:
- DE-A1- 4 122 494
- DE-U1- 29 707 307

## Beschreibung

Die Erfindung betrifft ein Verfahren und ein Gerät zur Beschaffenheitsuntersuchung von Holz und gegebenenfalls anderen säulenförmigen oder zylindrischen Abschnitten von Körpern.

Verfahren für die Untersuchung von Holz, insbesondere von lebendem Holz, um dessen Beschaffenheit zu untersuchen, sind bekannt; damit sollen insbesondere Fäulnis- und andere Prozesse frühzeitig detektiert werden, etwa aus Verkehrssicherheitsgründen.

Nicht invasive Verfahren umfassen hierbei die visuelle Analyse von Krone und Stamm. Im Inneren des Stammes sich entwickelnde oder bereits im fortgeschrittenen Zustand befindliche Fäulniszustände können visuell üblicherweise jedoch nicht ermittelt werden. Daher wurde zur Erlangung von Materialproben aus dem Inneren eines Baumes vielfach die Bohrkernentnahme eingesetzt, die als stark invasives Verfahren jedoch schädigenden Einfluss auf den Baum haben kann.

Um diesen Nachteil zu vermeiden, wurden weniger invasive Verfahren wie die Bohrwiderstandsmessung, die in der DE 3 501 841 beschrieben wird, entwickelt. Zur Untersuchung wird eine Bohrnadel einige Zentimeter tief im Wesentlichen senkrecht zur Stammachse in das Holz eindringen gelassen und der Widerstand der Bohrnadel beim definierten Einführen in den Stamm gemessen. Starke Abweichungen vom erwartungsgemäßen Widerstand oder ein Zusammenbrechen des mechanischen Widerstands indizieren einen Defekt, bedingt durch Fäulnis oder eine Aushöhlung. Obgleich mit diesem Verfahren qualitative Aussagen, insbesondere, wenn die Messung durch erfahrene Untersucher ausgeführt wird, erhalten werden, so ist doch die Erlangung quantitativer Aussagen schwierig, da die Bohrnadel beim Eindringen in weiches Material gleichmäßig vorgetrieben wird.

Eine Vorrichtung zur Durchführung von Bohrwiderstandsmessungen ist aus der DE 10 031 395 A1 bekannt, in der der Bohrwerkzeugantrieb in ein Becherteleskop einfahren gelassen wird, um eine verbesserte Führung des Bohrers bereit zu stellen. In der dort offenbarten Vorrichtung wird durch eine separate Antriebseinheit, die Drehung und Vorschub des Bohrwerkzeugs bewirkt, die Leistungsaufnahme gemessen und aufgezeichnet. Hierzu ist ein durch alle Becherböden des Becherteleskops laufendes Seil, das Vorschub-Zug-Seil, etwa über einen Seil-Wickel-Mechanismus mit einem Drehgeber verbunden oder mit einer optoelektronischen Registrierung von Abstands-Markierungen auf dem Seil gekoppelt. Die Anzahl der Becherböden erhöht folglich die Messungenauigkeit.

Auch aus der DE 41 22 494 A1 ist eine Vorrichtung zur Durchführung von Bohrwiderstandsmessungen bekannt. Die Vorrichtung weist ein Gehäuse auf und eine daraus ausfahrende, in das zu untersuchende Material eindringende Bohrnadel, die mit einem oder zwei Motoren vorgetrieben wird. Für die Materialdichtemessung wird die Leistungsaufnahme des Nadelantriebes erfasst und aufgezeichnet, während die Nadel auf einem Schlitten montiert mit gleichmäßigem Vorschub angetrieben wird. Der Vorschub ist insofern auch bei sich ändernder Materialbeschaffenheit konstant, hier muss daher die Leistung der Antriebseinheit erfasst, ausgelesen und der Messwert muss in Korrelation mit der Materialbeschaffenheit gesetzt werden.

DE 40 04 242 A1 offenbart ebenfalls ein Verfahren und eine Vorrichtung zur Untersuchung von Holzquerschnitten: Die Vorrichtung ist als einfaches Zusatzgerät für eine handelsübliche batteriebetriebene Handbohrmaschine konzipiert. Bei dieser Vorrichtung dient die Nadelspitze als Einzugshilfe, gleich einem Korkenzieher um in das Material einzudringen. Hier erfolgt eine Messung des Eindringwiderstands während des Einziehens der Prüfnadel, indem die Geschwindigkeit gemessen wird.

Ausgehend von diesem Stand der Technik besteht daher das Erfordernis, eine Vorrichtung zu schaffen, die es ermöglicht, durch eine minimal-invasive Prüfung von Holz, insbesondere von Baumstämmen, oder anderen säulenförmigen Körpern oder Körpern mit säulenförmigen Abschnitten, die nicht aus Holz, sondern aus anderen Werkstoffen beschaffen sind, mit apparativ geringst möglichem Aufwand, eine qualitative und möglichst quantitative Aussage über deren Beschaffenheit zu treffen. Dabei soll die während der Messung aufgenommene Information möglichst ohne Weiteres erfassbar sein, vorzugsweise ohne weitere Ablesegeräte.

Diese Aufgabe wird durch ein Handprüfgerät mit den Merkmalen des unabhängigen Anspruchs 1 gelöst.

Entsprechend ergibt sich die Aufgabe, ein verbessertes Prüfverfahren bereitzustellen, das es ermöglicht, *in-situ* und unter geringst möglichem Zeitaufwand eine qualitative und quantitative Aussage über die Beschaffenheit von Holz, insbesondere von Baumstämmen, oder anderen säulenförmigen Körpern oder Körpern mit säulenförmigen Abschnitten, die nicht aus Holz, sondern aus anderen Werkstoffen beschaffen sind, zu erhalten.

Diese Aufgabe wird durch ein Verfahren mit den Merkmalen des unabhängigen Anspruchs 7 gelöst.

Bevorzugte Ausführungsformen der Vorrichtung und des Verfahrens werden in den jeweiligen Unteransprüchen ausgeführt.

Ein erfindungsgemäßes Handprüfgerät dient der Untersuchung der Beschaffenheit von säulenförmigen oder zylindrischen Abschnitten von Objekten. Es umfasst eine Antriebsvorrichtung, die ein Bohrfutter hat, in das eine Bohrnadel eingelegt ist, die durch die Antriebsvorrichtung betätigt und dabei in ein Objekt eingetrieben werden kann. Das Handprüfgerät verfügt über eine teleskopierbare Führungsvorrichtung aus zwei Rohrabschnitten, wobei der innere Rohrabschnitt der kürzere ist und in einem längeren äußeren Rohrabschnitt axial verschiebbar angeordnet ist. An seinem Außenumfang weist der kürzere innere Rohrabschnitt eine längs angeordnete Messskala auf.

Das Teleskoprohr ist direkt oder indirekt durch den inneren Rohrabschnitt auf der Antriebsvorrichtung nicht drehend aufgesetzt. Weiter weisen sowohl der innere als auch der äußere Rohrabschnitt Führungsmittel wie Führungsbuchsen oder Hülsen auf, die sicher stellen, dass die Bohrnadel in den Teleskoprohrabschnitten zentral-axial geführt werden kann. Im Bohrfutter kann die Bohrnadel mit einer Klemmschraube fixiert werden; alternativ kann eine Befestigung durch ein an der Bohrnadel außen vorliegendes Gewinde erhalten werden.

Die Bohrnadel erstreckt sich zentral-axial von dem Bohrfutter durch den inneren Rohrabschnitt hindurch bis hin zu dem äußeren Rohrabschnitt, aus dem sie sich entweder heraus erstreckt oder mit dem sie bündig abschließt. Die Bohrnadel wird in den Rohrabschnitten des Teleskoprohres geführt; sie ist so angeordnet, dass sie sich beim Einschieben des inneren Rohrabschnitts (in einer Gebrauchsposition), verbunden mit einem manuellen Vorschieben der Antriebsvorrichtung, auf der der innere Rohrabschnitt sitzt, in dem äußeren Rohrabschnitt durch das Führungsmittel geführt in das zu untersuchende Objekt eintreiben lässt.

Damit wird erfindungsgemäß ein einfach zu handhabendes Prüfgerät bereitgestellt, das keinen Schrieb aufweist, weder einen elektronischen noch einen mechanischen. Es wird allein der axiale Penetrationswiderstand der rotierenden Bohrnadel bei Inbetriebnahme des Geräts erfasst, eine Torsionsleistung wird nicht gemessen.

Das erfindungsgemäße Handprüfgerät weist zwar eine im Inneren geführte und mit einer Antriebseinheit gleich dem einer Bohrmaschine angetriebene Nadel auf, die jedoch nicht gleichmäßig vorgeschoben wird, was manuell mittels der beiden Führungsrohre erfolgt. Je nach Struktur kann hier die Vorschubsgeschwindigkeit in Abhängigkeit der Materialeigenschaften variieren. Aufgrund dieser Vorgehensweise ist es dem Anwender sofort und ohne weitere Vorrichtungen erlaubt, die Unterschiede im Material zu erkennen und durch einfaches Ablesen der Messskala zu bewerten.

Im Allgemeinen erfordert hartes Material bei der Messung eine höhere Anpresskraft des Gerätes an das zu untersuchende Material als weiches; bei Hohlräumen ist keine Anpresskraft nötig. So wird durch das erfindungsgemäße Handprüfgerät die Nadel durch gleichmäßigen Druck in das Material eingebracht und es werden die Drehmomentsunterschiede in Abhängigkeit der Bohrtiefe erfasst.

Vorteilhaft kann, wenn eine Höhlung im Material erfasst wird, die Maschine nach Durchbohren der Restwand ausgeschaltet werden und es kann mit der stehenden Nadel durch Zurückziehen derselben die Restwandstärke an der Skala abgelesen und so vermessen werden. Durch den dickeren Nadelkopf lässt sich dabei auf einfache Weise ein deutlicher Unterschied zwischen Hohlraum und Bohrkanal erkennen.

Die Nadelführung des Handprüfgeräts kann über die Teleskoprohre mit innen liegenden Führungsbuchsen realisiert werden; im Bohrfutter wird die Nadel mit einer Klemmschraube fixiert. Alternativ kann eine Befestigung auch über ein Außengewinde an der Bohrnadel erfolgen.

Eine weitere Alternative, um eine Führung bereitzustellen, kann durch Führungsmittel erhalten werden, die an den von der Antriebsvorrichtung weg weisenden Enden des inneren und äußeren Rohrabschnitts vorliegen. Dazu können Hülsen eingesetzt werden. Eine Hülse an dem längeren äußeren Rohrabschnitt kann ein Spiral-Innengewinde aufweisen, dessen Form einem an der Nadel vorliegenden Außengewinde entspricht.

Vorteilhaft ist der kürzere innere Rohrabschnitt des Teleskoprohrs, über den selbiges mit der Antriebsvorrichtung in Kontakt steht, über eine einfache Hülse mit der Antriebsvorrichtung nicht drehend verbunden; eine solche Hülse kann einfach aufgesteckt sein. Sie dient als Distanzhalter und wird üblicherweise einen Innendurchmesser aufweisen, der dem Außendurchmesser der Bohrnadel angepasst ist.

Zur bequemen Handhabung des erfindungsgemäßen Handprüfgeräts kann dieses um seinen Außenumfang des äußeren Rohrabschnitts eine Griffhülse aufweisen. Eine solche Griffhülse ist besonders bequem handhabbar, wenn sie aus einem elastischen Material gefertigt ist. Die Griffhülse hilft, das Teleskoprohr zu führen und zu halten.

Weiter ist die an dem inneren Rohrabschnitt vorliegende Messskala, die sich über die gesamte Länge des inneren Rohrabschnitts erstrecken kann, zumindest aber an dem Längsabschnitt vorliegt, der zu der Antriebsvorrichtung hinweist, mit einer Skalierung ausgestattet, die dem Ablesen einer Einschublänge des inneren Rohrabschnitts in den äußeren Rohrabschnitt in Millimetern, Inch, Zoll oder Zentimetern aufweist.

So kann mit dem Handprüfgerät ein Prüfverfahren zur Untersuchung der Beschaffenheit von säulenförmigen oder zylindrischen Abschnitten von Objekten ausgeführt werden, das zunächst das Ausziehen des inneren aus dem äußeren Teleskoprohr vorsieht, gefolgt vom Ansetzen der Nadel, die sich aus dem äußeren Rohrabschnitt durch das Führungsmittel geführt heraus erstreckt, an dem zu untersuchenden Objekt. Bei diesem Objekt kann es sich um einen Baum handeln, es kann jedoch auch totes Holz oder es können andere Materialien untersucht werden.

Nunmehr wird die Antriebsvorrichtung in Gang gesetzt und die Nadel beginnt zu rotieren. Dabei wird die Nadel in das Objekt eingetrieben, wobei sich ein aus dem Objektmaterial resultierender ersten Widerstand ergibt, den die Nadel beim Eintreiben in das Objekt überwinden muss. Durch das Eintreiben der Nadel in das Objekt ergibt sich, dass die in der Hand gehaltene Antriebsvorrichtung axial in Richtung des Objekts vorgeschoben wird und dabei den inneren Rohrabschnitt des Teleskoprohrs in den äußeren Rohrabschnitt einschiebt. Wird die Nadel bei der Untersuchung eines Baums verwendet, so ändert sich der axiale Penetrationswiderstand der rotierenden Nadel abrupt, respektive sprunghaft. Diese Widerstandsänderung wird vom Anwender unmittelbar und deutlich wahrgenommen; in diesem Augenblick erfolgt das Ablesen der Einschublänge des inneren Rohrabschnitts in den äußeren Rohrabschnitt. So wird ein direktes Maß darüber erhalten, bis zu welcher Eindringtiefe die Nadel sich durch gesundes Holz bewegt hat, das einen höheren Penetrationswiderstand aufweist, als das weiche, faule Holz.

Das Handprüfgerät ist leicht transportabel und einfach gestaltet, es kann von jedermann bedient werden und bedarf keiner umständlichen Installation an einem zu untersuchenden Objekt, bei dem es sich vornehmlich um Holz, insbesondere um lebende Bäume handeln wird, die vielfach gerade in urbanen Gegenden eine Gefahr darstellen, wenn sie von Fäulnis befallen sind und an Straßen stehen.

So ist das erfindungsgemäße Handprüfgerät ein vorteilhaftes Mittel, um rasch und kostengünstig eine hohe Anzahl von Bäumen und Holzkonstruktionen sowie Holzmasten auf das Vorliegen von Fäulnis zu überprüfen und damit einen Beitrag zur Verkehrssicherheit zu leisten.

Weitere Ausführungsformen, sowie einige der Vorteile, die mit diesen und weiteren Ausführungsformen verbunden sind, werden durch die nachfolgende ausführliche Beschreibung deutlich und besser verständlich. Unterstützend hierbei ist auch der Bezug auf die Figur in der Beschreibung.

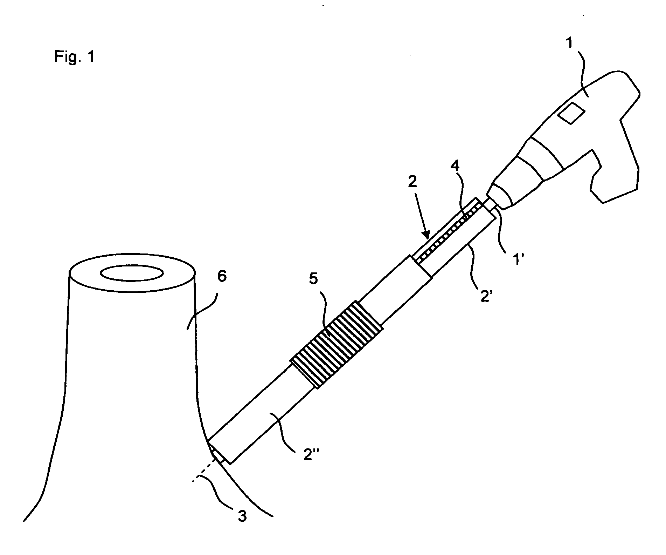

**Fig.** 1 zeigt eine Seitenansicht eines erfindungsgemäßen Handprüfgeräts.

**Fig. 1** zeigt ein erfindungsgemäßes Handprüfgerät, mit dem Holz, Holzmasten, insbesondere lebendes Holz, respektive Bäume auf ihre Beschaffenheit untersucht werden können, die sich etwa durch Änderungen der Dichte und der chemischen Zusammensetzung äußert. Der Begriff "Beschaffenheit" lässt sich an Hand eines Beispiels verdeutlichen: Ein Baumstamm zeigt im Querschnitt im Wesentlichen die bekannten Jahresringe, die beim Altern des Baumes entstehen und die sich etwa durch ihre Holzfaserzusammensetzung und deren Aufbau und Wassergehalt unterscheiden können, aber er kann beim kranken Baum auch zersetzte und in der Zersetzung begriffene Abschnitte umfassen, deren Zusammensetzung etwa auch Pilz und deren Zersetzungsprodukte - um lediglich beispielhaft eine Komponente zu nennen - enthalten kann.

Das Gerät hat keinen Schrieb, weder einen elektronischen noch einen mechanischen, es misst allein den axialen Penetrationswiderstand einer rotierenden Nadel beim Eindringen in das Objekt. Eine Torsionsleistung wird nicht erfasst. Die Axialkraft, die hierbei aufzubringen ist, wird vom Anwender mit der Hand gefühlt.

Damit dies einfach und sicher von jedem Anwender ausgeführt werden kann, ist das Handprüfgerät wie in **Fig. 1** gezeigt, gestaltet: Es verfügt über eine Antriebsvorrichtung 1, bei der es sich um eine handelsübliche Bohrmaschine 1 handeln kann. Die Antriebsvorrichtung hat ein Bohrfutter, in das eine Bohrnadel 3 eingesetzt werden kann. Diese wird durch die Antriebsvorrichtung angetrieben. Ferner verfügt das Handprüfgerät über eine Führung zum geführten Eindringen der Bohrnadel 3 in das zu untersuchende Objekt 6, vorliegend ein Baumstumpf, und eine Ablesevorrichtung 4, die zeigt, in welcher Tiefe das kranke Holz zu finden ist.

Bei der Führungsvorrichtung handelt es sich um ein Teleskoprohr 2, das einen inneren Rohrabschnitt 2' umfasst, der kürzer ist, als der ihn umgebende äußere Rohrabschnitt 2". Der innere Rohrabschnitt 2' ist in dem äußeren Rohrabschnitt 2" axial verschiebbar angeordnet und weist die Messskala 4 auf. Die Messskala 4 weist eine Bohrtiefenanzeige in Zentimeter, Zoll, Inch und Millimetereinteilung auf.

Wie zu sehen ist, wird die Bohrnadel 3, die vorliegend bereits in das Objekt 6 eingedrungen ist, an dem äußeren Rohrabschnitt 2" durch Führungsmittel 6 eingedrungen ist, an dem äußeren Rohrabschnitt 2" durch Führungsmittel geführt. Vorliegend erfolgt die Nadelführung über die Teleskoprohre mit innen liegenden Führungsbuchsen; im Bohrfutter wird die Nadel mit einer Klemmschraube fixiert.

So wird sichergestellt, dass die Nadel zentral-axial vom Bohrfutter der Antriebsvorrichtung 1 bis zu ihrem Ausgang aus dem äußeren Rohrabschnitt 2" geführt wird. Auch der innere Rohrabschnitt 2", figurativ nicht dargestellt, kann an seinem von der Bohrmaschine 1 wegweisenden Ende Führungsmittel aufweisen.

Die Hülsen, die als Führungsmittel dienen, können in einen Abschlussdeckel, auf den inneren und den äußeren Rohrabschnitt jeweils aufgesetzt sein kann, eingebettet sein. Im Übrigen kann der innere Rohrabschnitt nahezu massiv gestaltet sein und in seinem Inneren lediglich eine Längsbohrung mit einem Innendurchmesser aufweisen, der gerade größer ist als der Auβendurchmesser der Nadel. Das unmittelbar an dem Baum zu liegen kommende Führungsmittel des äußeren Rohrabschnitts kann mit einem Spiralgewinde ausgestattet sein, das bezüglich seiner Form dem Spiralgewinde der Nadel angepasst ist.

Ferner verfügt die erfindungsgemäße Handprüfvorrichtung, siehe Fig.1, über eine Griffhülse 5, die es ermöglicht, eine Hand zur Führung und zum Halten des äußeren Teleskoprohrs an diesem sicher und bequem aufzulegen.

So kann eine Messung zur Erfassung von Fäulnis in einem Baum ausgeführt werden, indem der äußere Rohrabschnitt des Teleskoprohrs mit seiner Austrittsöffnung für die Nadel an einen Baum angelegt wird, wobei der innere Rohrabschnitt bei Beginn der Messung ausgezogen vorliegt. Nun wird die Bohrmaschine eingeschaltet und die Nadel beginnt zu rotieren. Dabei wird die Bohrmaschine samt dem inneren Rohrabschnitt, der über eine Distanzangelegt ist, in den äußeren Rohrabschnitt hinein geschoben.

Hierbei muss ein Penetrationswiderstand, der durch das gesunde Holz bereitgestellt wird, überwunden werden. Analog verhält es sich, wenn andere Objekte als Bäume gemessen werden. Sobald sich die Materialzusammensetzung, und hiermit verbunden der Penetrationswiderstand, ändert, erfolgt eine sprunghafte Bewegung der Nadel tiefer in das Holz, respektive in das zu untersuchende Objektmaterial. Bei der Untersuchung von Bäumen sind hierbei selbst Jahresringe von Nadelbäumen als "Ruckeln" wahrnehmbar. In dem Moment, in dem sich der Penetrationswiderstand sprunghaft ändert, kann an dem inneren Rohrabschnitt an der Messskala abgelesen werden, wie weit diese bei dem vorher herrschenden Penetrationswiderstand in den äußeren Rohrabschnitt eingeschoben werden konnte.

Bei der Untersuchung von Holz lässt sich hierbei folglich ablesen, welcher Abschnitt des Holzes gesund ist und daher den höheren, meist zuerst erfassten Penetrationswiderstand aufbringt. Grundsätzlich lässt sich so auf einfache Art und Weise nahezu durch jedermann ermitteln, ob im Inneren des Baumes oder Holzes Fäulnis vorliegt. Damit ergänzt das erfindungsgemäße Handprüfgerät zu visuellen Baumkontrollen eine einfache und kostengünstige Untersuchung und kann daher dazu beitragen, erkrankte Bäume schnellstmöglich aufzufinden und damit zu verhindern, dass durch den erkrankten Baum ein Schaden entsteht. Vorteilhaft ist das erfindungsgemäße Verfahren dabei nur minimal invasiv, der Körper, der ein Stamm eines lebenden Baumes oder auch eine Betonsäule sein kann, wird daher nur insoweit geschädigt, als eine Bohrnadel in den Körper eingeführt wird; die Entnahme von Material in größerem Umfang wird vermieden und somit eine Materialschwächung oder Schädigung an der Untersuchungsstelle auf ein Mindestmaß begrenzt. Dennoch wird außer der qualitativen Aussage darüber, ob etwa Fäulnis vorliegt oder nicht, eine quantitative Aussage getroffen, in welcher Tiefe die Fäulnisschicht vorliegt und gegebenenfalls sogar, wie groß ihre Mächtigkeit ist.

## Patentansprüche

1. Handprüfgerät zur Untersuchung der Beschaffenheit von säulenförmigen oder zylindrischen Abschnitten von Objekten (6), umfassend
- eine Antriebsvorrichtung (1) mit einem Bohrfutter, in das eine Bohrnadel (3), die durch die Antriebsvorrichtung (1) antreibbar ist, aufgenommen ist,
- eine Führungsvorrichtung zum geführten Einführen der Bohrnadel (3) in das zu untersuchende Objekt (6),
**dadurch gekennzeichnet, dass**
die Führungsvorrichtung ein Teleskoprohr (2) ist,
das einen kürzeren inneren Rohrabschnitt (2') umfasst, der in einem längeren äußeren Rohrabschnitt (2") axial verschiebbar angeordnet ist und der an seinem Außenumfang eine längs angeordnete Messskala (4) aufweist, wobei das Teleskoprohr (2)
über den inneren Rohrabschnitt (2') auf die Antriebsvorrichtung (1) nicht drehend aufgesetzt ist,
wobei der innere und der äußere Rohrabschnitt (2',2") Führungsbuchsen oder Hülsen zum zentral-axialen Führen der Bohrnadel (3) in dem Teleskoprohr (2) aufweisen, die sich zentral-axial von dem Bohrfutter durch den inneren Rohrabschnitt (2') und durch den äußeren Rohrabschnitt (2") erstreckt,
und wobei die Bohrnadel (3) bei einem Einschieben des inneren Rohrabschnitts (2") in den äußeren Rohrabschnitt (2") durch das Führungsmittel geführt in das zu untersuchende Objekt (6) eintreibbar ist.

2. Handprüfgerät nach Anspruch 1,
**dadurch gekennzeichnet, dass**
- die Führungsbuchsen in dem inneren Rohrabschnitt (2') und dem äußeren Rohrabschnitt (2") vorliegende Führungsbuchsen sind,
- die Hülsen an den von der Antriebsvorrichtung (1) weg weisenden Enden der Rohrabschnitte (2',2") angeordnet sind, und/oder dass eine der Hülsen, die an dem äußeren Rohrabschnitt (2") vorgesehen ist, ein Spiral-Innengewinde aufweist, dessen Form der Form eines an der Bohrnadel (3) vorliegenden Außengewindes entspricht.

3. Handprüfgerät nach Anspruch 1 oder 2,
**dadurch gekennzeichnet, dass**
eine Hülse (1'), die mit dem inneren Rohrabschnitt (2') verbunden ist, über das Bohrfutter der Antriebsvorrichtung (1) nicht drehend aufgesetzt ist.

4. Handprüfgerät nach zumindest einem der vorstehenden Ansprüche,
**dadurch gekennzeichnet, dass**
um einen Außenumfang des äußeren Rohrabschnitts (2") eine Griffhülse, insbesondere eine Griffhülse (5) aus einem elastischen Material, zur führenden und haltenden Handhabung des Teleskoprohrs (2), angeordnet ist.

5. Handprüfgerät nach zumindest einem der vorstehenden Ansprüche,
**dadurch gekennzeichnet, dass**
die längs angeordnete Messskala (4) eine Skalierung zum Ablesen einer Einschublänge des inneren Rohrabschnitts (2') in den äußeren Rohrabschnitt (2") in Millimetern oder Zentimetern aufweist.

6. Handprüfgerät nach zumindest einem der vorstehenden Ansprüche
**dadurch gekennzeichnet, dass**
die Nadel (3) im Bohrfutter mit einer Klemmschraube festgelegt ist.

7. Verfahren zur Untersuchung der Beschaffenheit von säulenförmigen oder zylindrischen Abschnitten von Objekten (6) unter Verwendung eines Handprüfgeräts nach zumindest einem der Ansprüche 1 bis 6,
**umfassend die Schritte**
- Bereitstellen des Teleskoprohrs (2), das einen kürzeren inneren Rohrabschnitt (2') umfasst, in einem ausgezogenen Zustand, und
- Ansetzen der Bohrnadel (3), die sich aus dem äußeren Rohrabschnitt (2") durch das Führungsmittel geführt heraus erstreckt, an dem zu untersuchende Objekt (6),
- Betätigen der Antriebsvorrichtung (1) und rotatorisch Antreiben der Bohrnadel (3) und Eintreiben der Bohrnadel (3) in das Objekt (6) gegen einen ersten durch das Objekt (6) bereitgestellten Widerstand, dabei
- handbetätigtes axiales Verschieben der Antriebsvorrichtung (1) in Objektrichtung und dabei Einschieben des inneren Rohrabschnitts (2') In den äußeren Rohrabschnitt (2");
- Ablesen einer Einschublänge des inneren Rohrabschnitts (2') in den äußeren Rohrabschnitt (2"), sobald sich der ersten durch das Objekt (6) bereitgestellte Widerstand sprunghaft ändert.

## Claims

1. A manual testing device for examining the properties and condition of columnar or cylindrical sections of objects (6), comprising
- a drive device (1) having a drill chuck, with a drill needle (3) which can be driven by the drive device (1) being received into said drill chuck,
- a guide device for guided insertion of the drill needle (3) into the object (6) to be examined,
**characterised in that**
the guide device is a telescopic tube (2)
which comprises a short inner tube section (2') that is arranged in a long outer tube section (2") such that it can be moved axially therein and which comprises a longitudinally arranged measuring scale (4) on its outer perimeter, wherein the telescopic tube (2)
is mounted on the drive device (1) via the inner tube section (2') in a non-rotary manner,
wherein the inner and the outer tube sections (2', 2") comprise guide bushings or sleeves for guiding the drill needle (3) in the telescopic tube (2) in a centrally axial manner, said drill needle (3) extending from the drill chuck through the inner tube section (2') and through the outer tube section (2") in a centrally axial manner,
and wherein the drill needle (3) can be driven into the object (6) to be examined by means of the guide device when the inner tube section (2') is inserted into the outer tube section (2").

2. The manual testing device according to Claim 1,
**characterised in that**
- the guide bushings are guide bushings that are present in the inner tube section (2') and the outer tube section (2"),
- the sleeves are arranged at the ends of the tube sections (2', 2") that are facing away from the drive device (1), and/or that one of the sleeves which is provided at the outer tube section (2") comprises a spiral internal thread the shape of which corresponds to the shape of an external thread that is present on the drill needle (3).

3. The manual testing device according to Claim 1 or 2,
**characterised in that**
a sleeve (1') that is connected to the inner tube section (2') is mounted via the drill chuck of the drive device (1) in a non-rotary manner.

4. The manual testing device according to at least one of the preceding claims,
**characterised in that**
a gripping sleeve, more particularly a gripping sleeve (5) made of an elastic material, is arranged around an outer perimeter of the outer tube section (2") for the purpose of handling the telescopic tube (2) in a guiding and holding manner.

5. The manual testing device according to at least one of the preceding claims,
**characterised in that**
the longitudinally arranged measuring scale (4) comprises a scale for reading a length of insertion of the inner tube section (2') into the outer tube section (2") in millimetres or centimetres.

6. The manual testing device according to at least one of the preceding claims,
**characterised in that**
the needle (3) is located in the drill chuck by means of a clamping screw.

7. A method for examining the properties and condition of columnar or cylindrical sections of objects (6) by means of a manual testing device according to at least one of Claims 1 to 6,
comprising the steps of
- providing the telescopic tube (2) in an extracted state, said telescopic tube (2) comprising a short inner tube section (2'), and
- applying the drill needle (3) to the object (6) to be examined, said drill needle (3) extending out of the outer tube section (2") while being guided by the guide device,
- actuating the drive device (1) and driving the drill needle (3) in a rotary manner and driving the drill needle (3) into the object (6) against a first resistance provided by the object (6), therein
- axially moving the drive device (1) in the direction of the object in a manually actuated manner and therein inserting the inner tube section (') into the outer tube section (2"),
- reading a length of insertion of the inner tube section (2') into the outer tube section (2") as soon as the first resistance provided by the object (6) changes in an abrupt manner.

## Revendications

1. Testeur manuel pour l'analyse de l'état de sections en forme de colonne ou cylindriques d'objets (6), comprenant
- un dispositif d'entraînement (1) avec un mandrin dans lequel une aiguille pour perceuse (3) qui peut être entraînée par le dispositif d'entraînement (1) est reçue,
- un dispositif de guidage pour l'introduction guidée de l'aiguille pour perceuse (3) dans l'objet à analyser (6),
**caractérisé en ce que**
le dispositif de guidage est un tuyau télescopique (2)
qui comprend une section de tuyau interne plus courte (2') qui est disposée de manière à pouvoir coulisser axialement dans une section de tuyau externe plus longue (2") et qui présente une échelle de mesure (4) disposée en longueur à sa périphérie externe, dans lequel le tuyau télescopique (2) est posé de manière non rotative sur le dispositif d'entraînement (1) par le biais de la section de tuyau interne (2'),
dans lequel la section de tuyau interne et la section de tuyau externe (2', 2") présentent des douilles de guidage ou manchons pour le guidage axial centralement de l'aiguille pour perceuse (3) dans le tuyau télescopique (2) qui s'étend de manière axiale centralement du mandrin à travers la section de tuyau interne (2') et à travers la section de tuyau externe (2"),
et dans lequel l'aiguille pour perceuse (3) peut être enfoncée de manière guidée dans l'objet à analyser (6) lors d'une insertion de la section de tuyau interne (2") dans la section de tuyau externe (2") à travers le moyen de guidage.

2. Testeur manuel selon la revendication 1,
**caractérisé en ce que**
- les douilles de guidage sont des douilles de guidage présentes dans la section de tuyau interne (2') et la section de tuyau externe (2"),
- les manchons sont disposés sur les extrémités des sections de tuyau (2', 2") tournées à l'écart du dispositif d'entraînement (1), et/ou
qu'un des manchons qui est prévu sur la section de tuyau externe (2") présente un filetage interne en spiral dont la forme correspond à la forme d'un filetage externe présent sur l'aiguille pour perceuse (3).

3. Testeur manuel selon la revendication 1 ou 2,
**caractérisé en ce que**
un manchon (1') qui est relié à la section de tuyau interne (2') est posé de manière non rotative au-dessus du mandrin du dispositif d'entraînement (1).

4. Testeur manuel selon au moins une des revendications précédentes,
**caractérisé en ce que**
un manchon de saisie, notamment un manchon de saisie (5) en un matériau élastique, est disposé autour d'une périphérie externe de la section de tuyau externe (2") pour le maniement de guidage et de maintien du tuyau télescopique (2).

5. Testeur manuel selon au moins une des revendications précédentes,
**caractérisé en ce que**
l'échelle de mesure (4) disposée en longueur présente une mise à l'échelle pour la lecture d'une longueur d'insertion de la section de tuyau interne (2') dans la section de tuyau externe (2") en millimètres ou centimètres.

6. Testeur manuel selon au moins une des revendications précédentes,
**caractérisé en ce que**
l'aiguille (3) est fixée dans le mandrin avec une vis de serrage.

7. Procédé d'analyse de l'état de sections en forme de colonne ou cylindriques d'objets (6) en utilisant un testeur manuel selon au moins une des revendications 1 à 6, comprenant les étapes
- mise à disposition du tuyau télescopique (2) qui comprend une section de tuyau interne plus courte (2') dans un état déplié, et
- application de l'aiguille pour perceuse (3) qui s'étend de manière guidée hors de la section de tuyau externe (2") à travers le moyen de guidage sur l'objet à analyser (6),
- actionnement du dispositif d'entraînement (1) et entraînement rotatif de l'aiguille pour perceuse (3) et enfoncement de l'aiguille pour perceuse (3) dans l'objet (6) à l'encontre d'une première résistance mise à disposition à travers l'objet (6), ce faisant
- coulissement axial actionné manuellement du dispositif d'entraînement (1) dans la direction de l'objet et ce faisant, enfoncement de la section de tuyau interne (2') dans la section de tuyau externe (2") ;
- lecture d'une longueur d'insertion de la section de tuyau interne (2') dans la section de tuyau externe (2") dès que la première résistance mise à disposition à travers l'objet (6) varie brusquement.
